(19) Europäisches Patentamt
European Patent Office
Office européen des brevets

(11) Publication number: **0 018 830**
**B1**

# (12) EUROPEAN PATENT SPECIFICATION

(45) Date of publication of patent specification: **08.06.83**

(51) Int. Cl.³: **C 07 D 307/94,**
A 61 K 31/34

(21) Application number: **80301431.5**

(22) Date of filing: **01.05.80**

## (54) Spirobenzofuranones, their production and pharmaceutical compositions containing them.

(30) Priority: **04.05.79 JP 55082/79**
**25.06.79 JP 80551/79**

(43) Date of publication of application:
**12.11.80 Bulletin 80/23**

(45) Publication of the grant of the patent:
**08.06.83 Bulletin 83/23**

(84) Designated Contracting States:
**BE CH DE FR GB IT LI NL SE**

(56) References cited:
**EP - A - 0 003 084**

**Chemical Abstracts vol. 68, no. 21, 20 May 1968 Columbus, Ohio, USA J. A. DONNELLY et al. "Reactions of chrom-indogenides with dimethylsulfoxonium methylide" page 9219, columns 1 to 2, abstract no. 95626z**

(73) Proprietor: **Takeda Chemical Industries, Ltd.**
**27, Doshomachi 2-chome**
**Higashi-ku Osaka, 541 (JP)**

(72) Inventor: **Sugihara, Hirosada**
**5-604, 2 Minase 2-chome**
**Shimamotocho Mishima-gun, Osaka 618 (JP)**
Inventor: **Watanabe, Masazumi**
**4-54, Seiwadainishi 2-chome**
**Kawanishi Hyogo 666-01 (JP)**
Inventor: **Kawada, Mitsuru**
**25-3, Tsukaguchicho 4-chome**
**Amagasaki, Hyogo, 661 (JP)**
Inventor: **Imada, Isuke**
**18-10, Tsuruyamadai 4-chome**
**Izumi, Osaka 594 (JP)**

(74) Representative: **Lewin, John Harvey et al,**
**Elkington and Fife High Holborn House 52/54**
**High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 018 830 B1**

Courier Press, Leamington Spa, England

# Spirobenzofuranones, their production and pharmaceutical compositions containing them

This invention relates to novel spiro compounds, these compounds for use as medicines and intermediates for the production of medicines, and methods for producing these compounds.

A spirobenzofuranone derivative has been disclosed by J. A. Donnelly et al in Chem. Ind. (London) no. 33, 1967, pages 1402 to 1403.

The present invention provides a spirobenzofuranone compound of the formula:

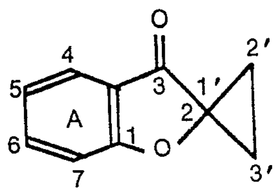

(I)

wherein Ring A represents a benzene ring which is substituted by one to four of carboxyl, $C_{2-6}$ alkoxycarbonyl, carbamoyl, $C_{1-4}$ alkylcarbamoyl, di-$C_{1-4}$ alkylamino-$C_{1-4}$ alkylcarbamoyl, N—$C_{1-4}$ alkylpyrrolidinyl-$C_{1-4}$ alkylcarbamoyl, ureido, $C_{1-4}$ alkylureido, thioureido, $C_{1-4}$ alkylthioureido, $C_{1-4}$ alkylthio, $C_{1-4}$ alkylsulphinyl, $C_{1-4}$ alkylsulphonyl, mono- or bis-($\beta$-hydroxyethyl)amino, aminomethyl, mono- or di-$C_{1-4}$ alkylaminomethyl, cyano and phenyl,

or a pharmaceutically acceptable salt thereof.

Referring to the above formula (I), the $C_{2-6}$ alkoxycarbonyl group is e.g. methoxycarbonyl, ethoxycarbonyl, *n*-propoxycarbonyl, iso-propoxycarbonyl or butoxycarbonyl.

Examples of $C_{1-4}$ alkylcarbamoyl include methylcarbamoyl, ethylcarbamoyl, *n*-propylcarbamoyl or iso-propylcarbamoyl.

Examples of $C_{1-4}$ alkylureido include methylureido, ethylureido, *n*-propylureido or iso-propylureido.

Examples of $C_{1-4}$ alkylthioureido include methylthioureido, ethylthioureido, *n*-propylthioureido or iso-propylthioureido.

The lower alkyl moieties of the $C_{1-4}$ alkylthio, $C_{1-4}$ alkylsulphinyl and $C_{1-4}$ alkylsulphonyl groups may, for example, be methyl, ethyl, *n*-propyl or isopropyl.

Examples of mono- or di-$C_{1-4}$ alkylaminomethyl include methylaminomethyl, ethylaminomethyl, dimethylaminomethyl or diethylaminomethyl.

These substituents of Ring A are present, up to 4 at the maximum, in substitutable positions on the ring, and may be the same or different. It is preferred that the benzene ring is mono-substituted, at its 5- or 7-position (5-position is more desirable), by carboxyl, $C_{1-4}$ alkylsulphonyl or mono- or bis-($\beta$-hydroxyethyl)amino, especially by carboxyl or methylsulphonyl.

The spiro compound (I) of the present invention is produced by, for instance, decarboxylating a compound of the formula:

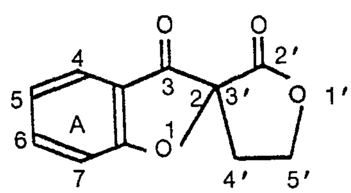

(II)

wherein Ring A is as defined hereinbefore.

This reaction is normally carried out in the presence of a catalyst which assists in decarboxylation. Preferred catalysts for this purpose include metal halide (e.g. sodium chloride, sodium bromide, sodium iodide, potassium bromide, potassium chloride or potassium iodide) and quaternary ammonium salts (e.g. tetramethylammonium bromide). The reaction temperature is normally from about 100°C to 200°C and preferably from about 140°C to 160°C, although the reaction may be conducted at higher or lower temperatures if it is desired to control the reaction velocity. Purging the reaction vessel with an inert gas (e.g. $N_2$ or argon) is sometimes effective in preventing side reactions and improving yields. This reaction is normally carried out in a suitable solvent. While any solvent that will not interfere with the reaction may be employed, it is normally advantageous to employ a solvent having a boiling point higher than the reaction temperature (e.g. dimethyl sulphoxide, N,N-dimethylformamide or hexamethylphosphoramide).

The spiro compound (I) may also be produced by converting at least one of the substituents of a compound of the formula:

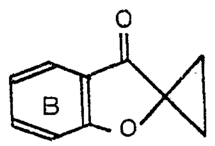

(III)

wherein Ring B represents a benzene ring which is substituted by one to four of amino, acetyl, halogen, carboxyl, $C_{2-6}$ alkoxycarbonyl, carbamoyl, $C_{1-4}$ alkylcarbamoyl, ureido, $C_{1-4}$ alkylureido thioureido, $C_{1-4}$ alkylthioureido, $C_{1-4}$ alkylthio, $C_{1-4}$ alkylsulphinyl, ($\beta$-hydroxyethyl)amino, aminomethyl and cyano.

The halogen atom in the above formula (III) may, for example, be chlorine or bromine and other substituents may have the same definitions as those defined in the formula (I).

As examples of the above reaction, there may be mentioned *per se* known conversion reactions in compliance with a substituent or substituents to be converted, such as alkylation, oxidation, reduction, haloform reaction, hydrolysis, and esterification. These reactions may be mentioned more especially as follows.

Compounds (I) having substituents on Ring A which are mono- or bis-($\beta$-hydroxyethyl)-amino, can be produced by alkylation of the compounds (III) substituted by corresponding amino groups, with ethylene chlorohydrin or ethylene oxide. Compounds (I), wherein the substituents on Ring A are carboxyl, are produced by subjecting an acetyl-substituted compound (III) to a haloform reaction with hypohalogenites (e.g. sodium hypochlorite or sodium hypobromite), or by subjecting a pyridinium salt obtained from the acetyl-substituted compound (III) with halogen (e.g. iodine) and pyridine to hydrolysis, or by subjecting to hydrolysis a cyano-substituted compound (III). A compound (I) substituted on Ring A by alkoxycarbonyl, can be produced by subjecting a carboxyl-substituted compound to esterification with alkyl halogenide or di-alkylsulphuric acid in the presence of alkali metal hydrogen carbonate (e.g. sodium hydrogen carbonate) or alkali metal carbonate (e.g. sodium carbonate). A compound (I), substituted by carbamoyl or substituted carbamoyl, is produced by subjecting a cyano-substituted compound (III) to hydrolysis with a base (e.g. sodium hydroxide or potassium hydroxide) in the presence of dimethylsulphoxide, or to a reaction with hydrogen peroxide under alkaline conditions, or by subjecting a carboxyl-substituted compound (III) or reactive derivative thereof to condensation with amines. This condensation may be carried out by reacting the carboxyl-substituted compound (III) with amines in the presence of a condensing agent (e.g. dicyclohexylcarbodiimide) and a suitable solvent (e.g. methylene chloride, pyridine or tetrahydrofuran). The reactive derivative of the carboxyl-substituted compound (III) is, for example, an acid halide, active ester (e.g. alkyl ester or *p*-nitrophenyl ester), mixed acid anhydride obtained from the carboxy compound (III) or an alkyl chlorocarbonate (e.g. methyl chlorocarbonate or isobutyl chlorocarbonate). A compound (I), substituted on Ring A by ureido, substituted ureido, thioureido, or substituted thioureido, is produced by subjecting an amino-substituted compound (III) to reaction with cyanic acid, isocyanic acid ester, thiocyanic acid or isothiocyanic acid ester. A cyano-substituted compound (I) is produced by subjecting a halo-substituted compound (III) or a diazonium salt derived from amino-substituted compound (III), to reaction with a metal cyanide (e.g. cuprous cyanide or sodium cyanide).

The desired compound (I) obtained in the foregoing manner can be isolated from the reaction mixture and purified by conventional procedures (e.g. distillation, recrystallization or column chromatography). According to the types of substituents on Ring A, the compound (I) may be isolated as pharmaceutically acceptable salts. For example, when an amino group (e.g. mono- or bis($\beta$-hydroxyethyl)amino or aminoalkylcarbamoyl) is present as the substituent, the compound (I) can be isolated as an acid addition salt (e.g. a mineral acid salt such as hydrochloride or hydrobromide, or an organic acid salt such as citrate, tartrate, maleate, fumarate, or oxalate), or when the substituent is a carboxyl group, the compound (I) can be isolated as an alkali metal salt (e.g. sodium salt or potassium salt). These salts and optical isomers are included in the scope of the present invention.

The spiro compounds (I) and pharmaceutically acceptable salts according to this invention are new compounds which exhibit gastric secretion inhibitive, antiinflammatory, analgesic and other actions in mammalian animals (e.g. man, rat, mouse, guinea-pig, dog and pig), for instance, and are of value as antiulcer, antiinflammatory and analgesic agents and as drugs for the management of peptic ulcer, acute or chronic gastritis, lumbago, arthritis and other diseases. Management of a peptic ulcer in accordance with the present invention includes both the prophylactic administration of the spiro compounds (I) to prevent the outbreak of an ulcer in an ulcer prone patient, as well as the treatment of an existing peptic ulcer. In such medicinal applications, each compound (I) can be safely administered orally or parenterally, either as it is or as formulated with pharmaceutically acceptable carriers or diluents known *per se* into suitable dosage forms such as tablets, powders, capsules, injections and suppositories. While the recommended dosage depends on the subject, condition, route of administration, etc., the normal oral dosage for the treatment of peptic ulcer or acute or chronic gastritis is about 1 mg. to 20 mg. as compound (I) per kg body weight per dose, to be given once to 3 times daily.

The starting compound (II) which is employed in the practice of this invention can be prepared by

the following synthesis route or any process analogous thereto.

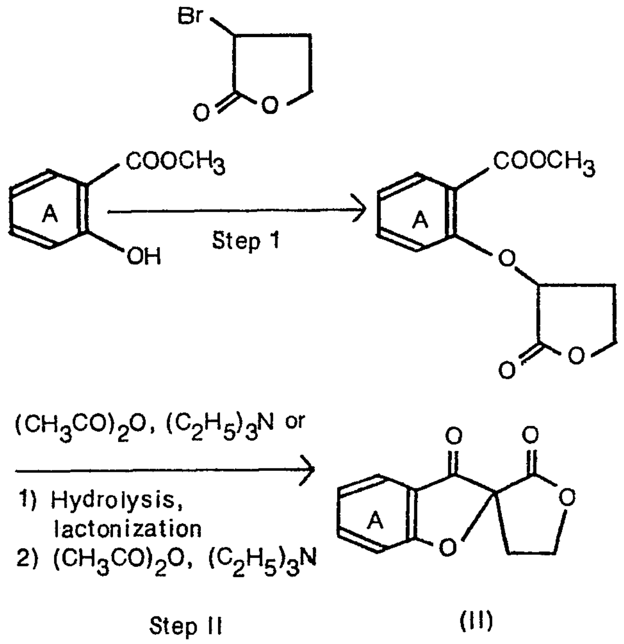


(II)

wherein Ring A is as defined hereinbefore.

The reaction of Step I is carried out by condensing a salicyclic acid derivative with $\alpha$-halogeno-$\gamma$-butyrolactone (e.g. $\alpha$-bromo-$\gamma$-butyrolactone) in the presence of a base (e.g. sodium hydride, sodium carbonate or potassium carbonate). The reaction of Step II comprises subjecting the product obtained by the reaction of Step I or after hydrolysis of the ester group, followed by lactonization of the product obtained by the reaction of Step I, to a Dieckmann condensation in the presence of an organic base (e.g. triethylamine, 1,4-diazabicyclo[2,2,2]octane or 1,8-diazabicyclo[5,4,0]-7-undecene).

A spiro compound (I) can be produced in one step by subjecting a product obtained by the reaction of Step I to a Dieckmann condensation in the presence of the base above-mentioned and the catalyst which assists decarboxylation also mentioned hereinbefore.

As the starting compounds (III), almost all the compounds (I) may be usable, and other compounds (III) may be prepared by the procedures mentioned hereinbefore. For example, an acetyl- or a halo- substituted compound (III) can be produced by decarboxylation of an acetyl- or a halo-substituted compound which is analogous to the compound (II) and is prepared by a similar procedure. An amino-substituted compound (III) can be produced by reduction of the corresponding nitro-substituted compound.

A compound of the formula:

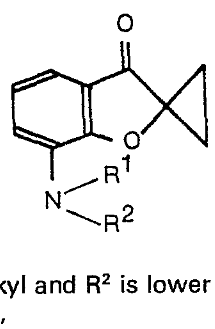

(IV)

wherein $R^1$ is hydrogen or lower alkyl and $R^2$ is lower alkyl, or $R^1$ and $R^2$, taken together with the adjacent N atom, form cyclic amino,

may be produced by subjecting a compound of the formula:

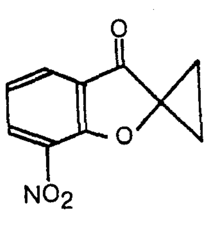

(V)

to reductive alkylation or subjecting a compound of the formula:

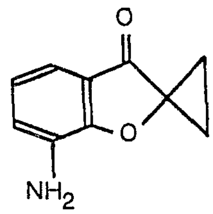

(VI)

to alkylation. Compounds IV are described and claimed in EP—A1—0 003 084 (Takeda).

The lower alkyls $R^1$ and $R^2$ may, for example, be $C_{1-4}$ alkyl (e.g. methyl, ethyl, *n*-propyl, isopropyl, *n*-butyl, isobutyl, sec-butyl or tert-butyl). The cyclic amino formed by $R^1$, $R^2$ together with the adjacent N atom may, for example, be 5- or 6-membered cyclic amino which may contain N or O as another hetero atom. Examples of 5- or 6- membered cyclic amino groups include pyrrolidinyl, piperidino, piperazinyl and morpholino, and the piperazinyl may be substituted by $C_{1-4}$ alkyl (e.g. methyl or ethyl), phenyl-$C_{1-4}$ alkyl (e.g. benzyl) or $C_{2-4}$ alkanoyl (e.g. acetyl or propionyl) at its 4-position.

The reductive alkylation may be carried out by subjecting the compound (V) to catalytic reduction with a catalyst such as platinum oxide, palladium or Raney nickel in the presence of a carbonyl compound (e.g. formalin, acetaldehyde or acetone) corresponding to the $R^1$ or $R^2$ group to be introduced. The reaction temperature is normally from 10°C to 100°C.

The alkylation of the compound (VI) may be carried out with an alkylating agent such as an alkyl halide (e.g. methyl iodide, ethyl iodide, ethyl bromide or isopropyl bromide), or by reductive alkylation mentioned above or reductive alkylation with a metal hydride (e.g. sodium cyanoborohydride).

A compound (IV) having a cyclic amino group may be produced by allowing the compound (VI) to react with, for example, $\omega,\omega$-dihalogenoalkane (e.g. 1,4-dibromobutane or 1,5-dibromopentane), bis (2-iodoethyl) ether, an N-alkyl derivative of $\beta,\beta'$-dihalogenoethylamine (e.g. N-methyl-$\beta,\beta'$-dichlorodiethylamine or N-ethyl-$\beta,\beta'$-diiododiethylamine) or an N-aralkyl derivative of the same (e.g. N-benzyl-$\beta,\beta'$-diiododiethylamine).

A compound of the formula:

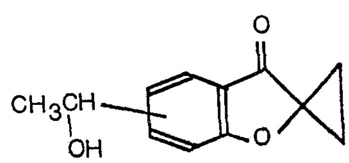

(VII)

may be produced by subjecting a compound of the formula:

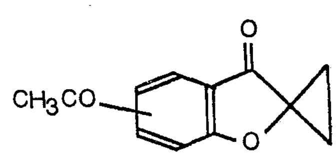

(VIII)

to reduction.

The position substituted by the 1-hydroxyethyl or acetyl group in the formula (VII) or (VIII) is preferably the 5- or 7- position.

The reduction is carried out by treating a compound (VIII) with sodium borohydride in a suitable solvent under mild conditions which do not attack the 3-carbonyl group.

The above compounds (IV) and pharmaceutically acceptable acid addition salts thereof, compounds (VII) and compounds obtained in Reference Examples 32, 33, 45 and 51 mentioned hereinafter have the same utility as that of the present compound (I) and are relatively less toxic. These compounds may be usable in accordance with the same manner as that of the compound (I).

The following pharmacological test, and reference and working examples are given to describe this invention in further detail but should by no means be considered to limit the scope of this invention.

*Pharmacological Test*

The pharmacological activity of the present compounds was assayed by a gastric-juice-secretion-inhibition test with rats, the results of which are as follows.

In accordance with the method described in "Gastroenterology" *5*, 43 (1945), inhibition of gastric-juice-secretion was evaluated by means of the pylorusligated rats.

Five each of male Sprague-Dawley rats (each weighing 100—130 g.) were used for the control and five test groups. Each animal was deprived of food for 18 hours before the test, except drinking

water. The pylorus of each animal was ligated under anesthetizing with ether, then the animals of the test groups were administered with each test compound at the dose of 50 mg./kg. intraduodenally. Three hours after the ligation, the animals were sacrificed. Gastric secretions of the tested animals were collected and centrifuged for 10 minutes (3,500 r.p.m.), and the volume of gastric juice was measured. The result is as shown in the following table.

The ICR-type mice in groups of five animals were administered orally with these compounds at the dose of 500 mg./kg. to examine acute toxicity. No mouse was found dead during 7 days in any groups.

Inhibition of Gastric-Juice Secretion in Rats

| Compound | Dose (i.d. mg./kg.) | Inhibition of Secretion (%) |
|---|---|---|
| 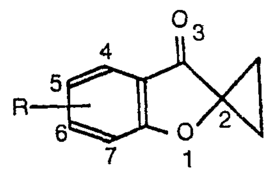 | | |
| 5—$N(CH_2CH_2OH)_2$ | 50 | 58 |
| 5—$NHCONHCH_3$ | 50 | 59 |
| 5—$NHCONH_2$ | 50 | 53 |
| 7—$N(C_2H_5)_2$ | 50 | 73 |
| 7–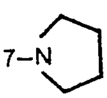 | 50 | 71 |

Reference Example 1

25 g. of β-bromo-γ-butyrolactone were added dropwise to a mixture of 15.2 g. of methyl salicylate, 12 g. of sodium hydroxide and 150 ml. of N,N-dimethylformamide under ice-cooling. The mixture was stirred at room temperature for 28 hours. The reaction mixture was made acidic by the addition of dilute hydrochloric acid and extracted with ethyl acetate. The extract was washed with water, dried and concentrated under reduced pressure. The residue was dissolved in 30 ml. of methanol, then 150 ml. of a 20% aqueous solution of sodium hydroxide was added dropwise and the solution was stirred at 55°C for 30 minutes. The reaction mixture was made acidic with 60 ml. of concentrated hydrochloric acid; the resulting precipitate (salicyclic acid) was filtered off and the filtrate was extracted with ethyl acetate. The extract was washed with water, dried and concentrated under reduced pressure.

The residue was dried *in vacuo* over phosphorus pentoxide for 24 hours, after which it was recrystallized from ethyl acetate-*n*-hexane (2:1). By the above procedure there was obtained 8.0 g of α-[(2-carboxyphenyl)oxy]-γ-butyrolactone as colourless needles melting at 113—115°C. (Determined by Hot-Plate method; in all the examples hereinafter, the same method was applied to determination of melting points).

Elemental analysis, for $C_{11}H_{10}O_5$
Calcd.: C, 59.46; H, 4.54
Found: C, 59.21; H, 4.51

Reference Example 2

Using 18.7 g of methyl 5-chlorosalicylate, the procedure of Reference Example 1 was repeated to obtain 9.3 g of α-[(2-carboxy-4-chlorophenyl)oxy]-γ-butyrolactone as colourless needles melting at 159—160.5°C.

Elemental analysis, for $C_{11}H_9ClO_5$
Calcd.: C, 51.48; H, 3.53; Cl, 13.82
Found: C, 51.22; H, 3.50; Cl, 13.70

Reference Example 3

To a solution of 22 g of methyl 3,5-dichlorosalicylate in 200 ml of dimethylformamide and 100 ml of toluene was added 5.8 g of sodium hydride (50% suspension in Bayol 85).

Then, under ice-cooling, a solution of 25 g of $\alpha$-bromo-$\gamma$-butyrolactone in 30 ml of toluene was added dropwise. The mixture was stirred at room temperature for 36 hours, after which time it was diluted with a small amount of water and distilled under reduced pressure to remove the solvent.

60 ml of a 20% aqueous solution of sodium hydroxide was added to the residue and the mixture was stirred at 50—60°C for one hour. The reaction mixture was made acidic with 40 ml of concentrated hydrochloric acid. The aqueous solution was extracted with ethyl acetate, washed with water, dried and distilled under reduced pressure to remove the solvent. The residue was dried over phosphorus pentoxide at 50°C for 12 hours, after which it was recrystallized from ethyl acetate. By the above procedure 14 g of $\alpha$-[(2-carboxy-4,6-dichlorophenyl)oxy]-$\gamma$-butyrolactone were obtained as colourless crystals melting at 117—120°C.

Elemental analysis, for $C_{11}H_8Cl_2O_5$
Calcd.: C, 45.38; H, 2.77
Found: C, 45.43; H, 2.66

Reference Example 4

43 g of $\alpha$-bromo-$\gamma$-butyrolactone were added under cooling with ice to a mixture of 34 g of methyl 5-acetylsalicylate, 29 g of anhydrous potassium carbonate and 350 ml of acetone, and the mixture was then refluxed for 15 hours. After cooling, the acetone was distilled off and 10% methanolic sodium hydroxide was added to the residue for hydrolysis. The reaction mixture was made acidic with hydrochloric acid and extracted with ethyl acetate. The extract was washed with water, dried over anhydrous sodium sulphate and distilled to remove the solvent. The residue was dissolved in dioxane (300 ml)-benzene (200 ml) and the solution was refluxed in the presence of $p$-toluenesulphonic acid (30 g), with the resulting water being continuously distilled off. The solvent was distilled off and the residue was diluted with water and extracted with ethyl acetate. The extract was washed with water, dried and concentrated to remove the solvent. The residue was recrystallized from ethyl acetate. By the above procedure $\alpha$-[(2-carboxy-4-acetylphenyl)oxy]-$\gamma$-butyrolactone was obtained as colourless needles, m.p. 155—158°C.

Yield 17 g.
Elemental analysis, for $C_{13}H_{12}O_6$
Calcd.: C, 59.09; H, 4.58
Found: C, 58.98; H, 4.48

Reference Example 5

51 g. of methyl 4-acetylamino-5-chloro-2-hydroxybenzoate and 36.8 g. of anhydrous potassium carbonate were suspended in 350 ml. of N,N-dimethylformamide. 55 g. of $\alpha$-bromo-$\gamma$-butyrolactone were added to the suspension, and the mixture was stirred at 60°C for 12 hours. The solvent was evaporated off under reduced pressure. The residue was diluted with water and extracted with ethyl acetate. The extract was washed with water, dried and concentrated to remove the solvent. The residue was dissolved in chloroform, and subjected to column chromatography on silica gel, using chloroform as the eluent. The product was recrystallized from methanol. By the above procedure $\alpha$-[(5-acetylamino-4-chloro-2-methoxycarbonylphenyl)oxy]-$\gamma$-butyrolactone was obtained as pale yellow prisms, m.p. 118—119°C.

Yield 32 g.
Elemental analysis, for $C_{14}H_{14}O_6NCl$
Calcd.: C, 51.31; H, 4.31; N, 4.27
Found: C, 51.24; H, 4.26; N, 4.16

Reference Example 6

63 g. of methyl 4-acetylamino-2-hydroxybenzoate were reacted in the same manner as in Reference Example 5. The product was subjected to column chromatography on silica gel and separated into two fractions. The crystals obtained from the first fraction were recrystallized from methanol to give 6-acetylamino-4',5'-dihydrospiro[benzo[b]-furan-2(3H),3'(2'H)-furan]-2',3-dione as colourless plates, m.p. 220—234°C.

Yield 1.4 g.
Elemental analysis, for $C_{13}H_{11}O_5N$
Calcd.: C, 59.77; H, 4.24; N, 5.36
Found: C, 59.71; H, 4.21; N, 5.28

From the second fraction $\alpha$-[(5-acetylamino-2-methoxycarbonylphenyl)oxy]-$\gamma$-butyrolactone was obtained as a pale yellow oil. Yield 35 g. This oily product can be subjected to the subsequent reaction step without further purification.

NMR($CDCl_3$)$\delta$: 2.10(3H, s, $NCOCH_3$), 2.65(2H, m, $CH_2$), 3.83(3H, s, $COOCH_3$), 4.45(2H, m, $OCH_2$), 4.98(1H, t, OCHCO), 7.09(1H, d, aromatic ring H), 7.66(1H, s, aromatic ring H), 7.73(1H, d, aromatic ring H).

Reference Example 7

3.04 g. of methyl salicylate were reacted with $\alpha$-bromo-$\gamma$-butyrolactone in the same manner as the corresponding step in Reference Example 4. The product was recrystallized from methanol to afford 3.3 g. of $\alpha$-[(2-methoxycarbonylphenyl)oxy]-$\gamma$-butyrolactone as colourless needles melting at 62—87°C.

Elemental analysis, for $C_{12}H_{12}O_5$
Calcd.: C, 61.01; H, 5.12
Found: C, 60.98; H, 4.99

Reference Example 8

A mixture of 1.3 g. of $\alpha$-[(2-carboxyphenyl)oxy-$\gamma$-butyrolactone, 15 ml. of acetic anhydride and 3 ml. of triethylamine was stirred in a nitrogen gas stream at 140°C for 3.5 hours, at the end of which time the solvents were distilled off under reduced pressure. Column chromatography was carried out on the residue using 32.5 g. of silica gel and carbon tetrachlorideacetone (10:1). The fraction corresponding to the contemplated compound was taken, concentrated under reduced pressure and recrystallized from $n$-hexane-ethyl acetate (3:1). By the above procedure 633 mg. of 4',5'-dihydro-spiro[benzo[b]-furan-2(3H), 3'(2'H)-furan]-2',3-dione were obtained as colourless needles melting at 111—111.5°C.

Elemental analysis, for $C_{11}H_8O_4$
Calcd.: C, 64.70; H, 3.95
Found: C, 64.74; H, 3.70

Reference Examples 9—11

The following compounds were produced by procedures similar to that described in Reference Example 8.

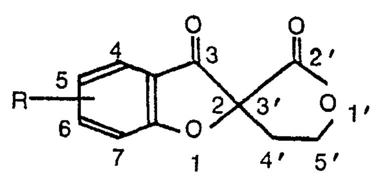

| Reference Example No. | Compound R | m.p. (°C) | Elemental analysis: Molecular formula | Upper rank: Calcd. Lower rank: Found — C | H |
|---|---|---|---|---|---|
| 9 | 5—Cl | 132.5—133 | $C_{11}H_7ClO_4$ | 55.36<br>55.49 | 2.96<br>2.79 |
| 10 | 5—Cl, 7—Cl | 157—159 | $C_{11}H_6Cl_2O_4$ | 48.38<br>48.47 | 2.21<br>2.14 |
| 11 | 5—$COCH_3$ | 132—134 | $C_{13}H_{10}O_5$ | 63.41<br>63.57 | 4.09<br>4.02 |

Reference Example 12

A mixture of 23 g. of $\alpha$-[(acetylamino-4-chloro-2-methoxycarbonylphenyl)oxy]-$\gamma$-butyrolactone, 46 ml. of triethylamine and 230 ml. of acetic anhydride was heated at 120°C for 5 hours. The solvents

were evaporated off under reduced pressure, then the residue was poured into ice-water. The precipitating crystals were collected by filtration, washed with water and dried, followed by recrystallization from ethyl acetate to give 6-diacetylamino-5-chloro-4',5'-dihydrospiro[benzo[b]furan-2(3H), 3'(2'H)-furan]-2',3-dione melting at 181—185°C. Yield 6.8 g.

Elemental analysis, for $C_{15}H_{12}O_6NCl$
Calcd.: C, 59.40; H, 4.32; N, 4.62
Found: C, 59.49; H, 4.21; N, 4.34

Reference Example 14

1.1 g. of α-[(2-methoxycarbonylphenyl)oxy]-γ-butyrolactone was treated as in Reference Example 8 and the product was recrystallized from ethyl acetate-*n*-hexane. By the above procedure 4',5'-dihydrospiro[benzo[b]furan-2(3H,3'(2'H)-furan]-2',3-dione was obtained as colourless needles, m.p. 111—111.5°C. Yield 330 mg.

Reference Example 15

A mixture of 0.35 ml. of nitric acid (d = 1.42) and 0.36 ml. of concentrated sulphuric acid was added to a solution of 0.408 g. of 4',5'-dihydrospiro[benzo[b]furan-2(3H),3'(2'H)-furan-2',3-dione in 3 ml. of concentrated sulphuric acid, dropwise under ice-cooling, and the mixture was stirred for 2 hours. The reaction mixture was poured into ice-water and the precipitated crystals were collected by filtration, washed with water, dried and recrystallized from ethyl acetate. By the above procedure colourless needles of 4',5'-dihydro-5-nitrospiro[benzo[b]furan-2(3H),3'(2'H)-furan]-2',3-dione were obtained, m.p. 199—200°C.

Elemental analysis, for $C_{11}H_7NO_6$
Calcd.: C, 53.02; H, 2.83; N, 5.62
Found: C, 52.89; H, 2.65; N, 5.55

Reference Examples 16—20

The following compounds were produced in a similar manner to the corresponding step in Reference Example 4.

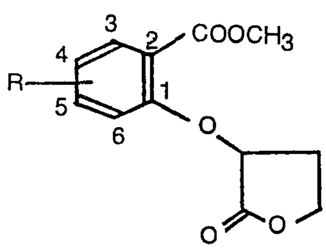

| No. | Compound R | m.p. (°C) | Molecular formula | Elemental analysis Upper rank: Calcd. Lower rank: Found C | H |
|---|---|---|---|---|---|
| 16 | 4—Ph | 112—114 | $C_{18}H_{16}O_5$ | 69.22<br>69.41 | 5.16<br>5.07 |
| 17 | 4—COPh | 98—102 | $C_{19}H_{16}O_6$ | 67.05<br>67.32 | 4.75<br>4.77 |
| 18 | 4—$COC_2H_5$ | 109—111 | $C_{15}H_{16}O_6$ | 61.64<br>61.63 | 5.52<br>5.56 |
| 19 | 4—$SCH_3$ | 60—63 | $C_{13}H_{14}O_5S$ | 55.30<br>55.09 | 5.00<br>4.93 |
| 20 | 6—$COCH_3$ | oil | $C_{14}H_{14}O_6$ | 60.43<br>60.21 | 5.07<br>5.02 |

(Ph represents phenyl)

Reference Example 21

α-[(2-Methoxycarbonyl-4-methylthiophenyl)oxy]-γ-butyrolactone (1.128 g.) was dissolved in dichloromethane (24 ml.) and *m*-chloro-perbenzoic acid (1.72 g.) was added to the solution in small portions. After stirring for one hour, the mixture was extracted with chloroform. The extract was washed with an aqueous solution of sodium hydrogen carbonate, an aqueous solution of sodium hydrosulphite and water in the order mentioned, and dried. The solvent was evaporated off under reduced pressure and the residue was recrystallized from ethyl acetate-hexane to give α-[(2-methoxycarbonyl-4-methylsulphonylphenyl)oxy]-γ-butyrolactone (1.089 g.) as colourless needles melting at 102—105°C.

Elemental analysis, for $C_{13}H_{14}O_7S$
Calcd.: C, 49.68; H, 4.49
Found: C, 49.67; H, 4.42

Reference Examples 22—26

The following compounds were obtained by a procedure similar to that of Reference Example 8.

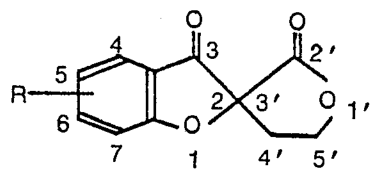

| No. | Compound R | m.p. (°C) | Elemental analysis Molecular formula | Upper rank: Calcd. Lower rank: Found C | H |
|---|---|---|---|---|---|
| 22 | 5—Ph | 193—195 | $C_{17}H_{12}O_4$ | 72.85<br>72.82 | 4.32<br>4.19 |
| 23 | 5—COPh | 197—200 | $C_{18}H_{12}O_5$ | 70.13<br>69.95 | 3.92<br>3.78 |
| 24 | 5—$COC_2H_5$ | 163—165 | $C_{14}H_{12}O_5$ | 64.61<br>64.70 | 4.65<br>4.57 |
| 25 | 5—$SCH_3$ | 100—102 | $C_{12}H_{10}O_4S$ | 57.59<br>57.82 | 4.03<br>3.90 |
| 26 | 5—$SO_2CH_3$ | 220—226 | $C_{12}H_{10}O_6S$ | 51.06<br>50.97 | 3.57<br>3.52 |

(Ph represents phenyl)

Reference Example 27

A mixture of 1.75 g. of 4',5'-dihydrospiro[benzo[b]furan-2(3H),3'(2'H)-furan]-2',3-dione, 552 mg. of sodium chloride and 9 ml. of dimethylsulphoxide was stirred in a nitrogen gas stream at 155°C for 2 hours. The reaction mixture was poured into ice-water (about 150 ml.) and the precipitate was recovered by filtration, washed with water and recrystallized from ethanol-water (3:2). By the above procedure 1.21 g. of spiro[benzo[b]furan-2(3H), 1'-cyclopropane]-3-one was obtained as colourless needles melting at 89—90.5°C.

Elemental analysis, for $C_{10}H_8O_2$
Calcd.: C, 74.99; H, 5.03
Found: C, 74.71; H, 4.96

Reference Examples 28—35

The following compounds were produced by a procedure similar to that described in reference Example 27.

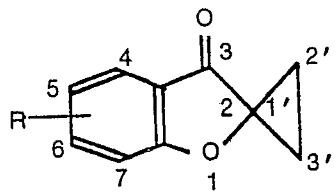

| No. | Compound R | Melting Point (°C) | Elemental Analysis: Molecular formula | Upper rank: Calcd. Lower rank: Found C | H | N |
|---|---|---|---|---|---|---|
| 28 | 5—Cl | 120—121 | $C_{10}H_7ClO_2$ | 61.71<br>61.68 | 3.63<br>3.50 | |
| 29 | 5—Cl<br>7—Cl | 116—118 | $C_{10}H_6Cl_2O_2$ | 52.43<br>52.65 | 2.64<br>2.61 | |
| 30 | 5—$NO_2$ | 107—110 | $C_{10}H_7NO_4$ | 58.54<br>58.85 | 3.44<br>3.50 | 6.83<br>6.68 |
| 31 | 5—$COCH_3$ | 100—103 | $C_{12}H_{10}O_3$ | 71.28<br>71.07 | 4.99<br>4.82 | |
| 32 | 5—COPh | 89—91 | $C_{17}H_{12}O_3$ | 77.26<br>77.30 | 4.58<br>4.33 | |
| 33 | 5—$COC_2H_5$ | 75—77 | $C_{13}H_{12}O_3$ | 72.21<br>72.16 | 5.59<br>5.52 | |
| 34 | 6—NHAc | 171—178 | $C_{12}H_{11}O_3N$ | 66.35<br>66.30 | 5.10<br>5.00 | 6.45<br>6.20 |
| 35 | 5—Cl,<br>6—NHAc | 185—188 | $C_{12}H_{10}O_3NCl$ | 57.27<br>57.03 | 4.01<br>3.86 | 5.57<br>5.46 |

(Ac represents acetyl).

Reference Example 36

0.94 g. of spiro[benzo[b]furan-2(3H), 1′-cyclopropane]-3-one was dissolved in 30 ml. of acetic anhydride and, at 60—70°C, 5.6 g. of copper nitrate were added. The solution was stirred overnight. The reaction mixture was poured into ice-water and extracted with ethyl acetate. The extract was washed with water, dried and distilled to remove the solvent. The residue was fractionated by column chromatography on silica gel into two fractions.

(1) The first fraction was recrystallized from ethyl acetate-*n*-hexane to yield 5-nitrospiro[benzo[b]furan-2(3H), 1′-cyclopropane]-3-one as colourless prisms melting at 107—110°C.

Elemental analysis, for $C_{10}H_7NO_4$
Calcd.: C, 58.54; H, 3.44; N, 6.83
Found: C, 58.85; H, 3.50; N, 6.68

(2) The second fraction was recrystallized from ethyl acetate-hexane to yield 7-nitrospiro[benzo[b]furan-2(3H), 1′-cyclopropane]-3-one as colourless needles melting at 131—134°C.

Elemental anaylsis, for $C_{10}H_7NO_4$
Calcd.: C, 58.54; H, 3.44; N, 6.83
Found: C, 58.42; H, 3.37; N, 6.65.

Reference Example 37

Spiro[benzo[b]furan-2(3H),1'-cyclopropane]-3-one (7.0 g.) was added in small portions to fuming nitric acid (70 ml.) previously cooled to −50°C to −60°C. After stirring for 20 minutes, the reaction mixture was poured into ice-water and the precipitated crystals were collected by filtration, washed with water and recrystallized from ethanol. By the above procedure 5-nitrospiro[benzo[b]-furan-2(3H),1'-cyclopropane]-3-one was obtained as colorless prisms, m.p. 107—110°C. Yield 7.3 g. This product was in good agreement with the crystals obtained in Reference Example 36.

The mother liquor resulting from the recrystallization was subjected to column chromatography on silica-gel for purification, then recrystallized from methanol to afford 5,7-dinitrospiro[benzo[b]furan-2-(3H),1'-cyclopropane]-3-one as pale yellow needles melting at 158—161°C.

Elemental analysis for $C_{10}H_6O_6N_2$
Calcd.: C, 48.01; H, 2.42; N, 11.20
Found: C, 48.03; H, 2.33; N, 11.01

Reference Example 38

A solution of 5-nitrospiro[benzo[b]furan-2(3H),1'-cyclopropane]-3-one (7.2 g.) in ethanol was stirred in the presence of platinum dioxide and in a hydrogen gas stream. After the hydrogen absorption ceased, the catalyst was filtered off and a small amount of HCl-diethyl ether was added to the residue, followed by recrystallization from ethanol. By the above procedure 5-aminospiro[benzo[b]furan-2(3H),1'-cyclopropane]-3-one hydrochloride was obtained as light-brown needles melting at 139—142°C.

Elemental analysis, for $C_{10}H_{19}O_2N{\cdot}HCl$
Calcd.: C, 56.75; H, 4.76; N, 6.62
Found: C, 56.67; H, 4.83; N, 6.67

Reference Example 39

7-Nitrospiro[benzo[b]furan-2(3H),1'-cyclopropane]-3-one was reacted in the same manner as in Reference Example 38 and the reaction product was recrystallized from ethanol. By the above procedure 7-aminospiro[benzo[b]furan-2(3H),1'-cyclopropane]-3-one was obtained as pale brown crystals melting at 135.8°C.

Elemental analysis, for $C_{10}H_{19}O_2N$
Calcd.: C, 68.56; H, 5.18; N, 8.00
Found: C, 68.42; H, 5.11; N, 7.74

Reference Example 40

250 mg. of 5,7-dinitrospiro[benzo[b]furan-2(3H),1'-cyclopropane]-3-one; 50 mg. of platinum dioxide and 20 ml. of ethanol were stirred in a stream of hydrogen for 1.25 hour under atmospheric pressure. Oxalic acid was added to the reaction mixture, and the catalyst was removed by filtration. The filtrate was concentrated under reduced pressure until its volume became about 3 ml. Ether was added to the concentrate, and the resulting powder was collected by filtration. The powder was dissolved in ethanol. To the ethanolic solution activated charcoal was added for decoloration, followed by addition of ether. The precipitating powder was collected by filtration to obtain 5,7-diaminospiro[benzo[b]furan-2(3H),1'-cyclopropane]-3-one.1/2 oxalate:monohydrate as a yellowish brown powder.

Elemental analysis, for $C_{10}H_{10}O_2N_2{\cdot}\frac{1}{2}(COOH)_2{\cdot}H_2O$
Calcd.: C, 52.17; H, 5.17; N, 11.06
Found: C, 52.12; H, 4.69; N, 10.87

The use of hydrochloric acid in place of oxalic acid in the above procedure gives 5,7-diaminospiro[benzo[b]furan-2(3H),1'-cyclopropane]-3-one hydrochloride·monohydrate melting at a temperature not lower than 300°C.

Elemental analysis, for $C_{10}H_{10}O_2N_2{\cdot}HCl{\cdot}H_2O$
Calcd.: C, 49.08; H, 5.35; N, 11.45
Found: C, 48.80; H, 5.13; N, 11.64

Reference Example 41

0.8 g. of potassium hydroxide were added to a solution of 1.09 g. of 6-acetylaminospiro[benzo[b]furan-2(3H),1'-cyclopropane]-3-one in 50 ml. of methanol, and the mixture was refluxed for 0.5 hour. The solvent was evaporated off under reduced pressure. Water was added to the residue and the precipitating crystals were collected by filtration, washed with water and dried. The crystals were recrystallized from methanol to obtain 6-aminospiro[benzo[b]furan-2(3H),1'-cyclopropane]-3-one as colourless prisms melting at 188—189°C.

Elemental analysis, for $C_{10}H_9O_2N$
Calcd.: C, 68.56; H, 5.18; N, 8.00
Found: C, 68.34; H, 5.05; N, 7.88

Reference Example 42

6-Acetylamino-5-chlorospiro[benzo[b]furan-2(3H),1'-cyclopropane]-3-one (1.8 g.) was reacted in the same manner as in Example 41 and the reaction product was recrystallized from methanol. By the above procedure 6-amino-5-chlorospiro[benzo[b]furan-2(3H),1'-cyclopropane]-3-one was obtained as yellow plates, m.p. 201°C. Yield 1.5 g.

Elemental analysis, for $C_{10}H_8O_2NCl$
Calcd.: C, 57.29; H, 3.85; N, 6.68
Found: C, 57.24; H, 3.74; N, 6.67

Reference Example 43

5-Aminospiro[benzo[b]furan-2(3H),1'-cyclopropane]-3-one (0.747 g.) and calcium carbonate (0.47 g.) were suspended in a mixture of carbon tetrachloride (20 ml) and methylene chloride (5 ml.). The suspension was cooled to −17°C, then bromine (0.22 ml.) was added thereto dropwise, followed by stirring for 45 minutes. The reaction mixture was poured into ice-water, then extracted with ethyl acetate. The extract was washed with water and dried. The solvent was evaporated off, and the residue was recrystallized from ethanol-water. By the above procedure 5-amino-4-bromospiro[benzo[b]furan-2(3H),1'-cyclopropane]-3-one was obtained as yellow needles melting at 167—170°C. Yield 0.6 g.

Elemental analysis, for $C_{10}H_8O_2NBr$
Calcd.: C, 47.27; H, 3.19; N, 5.51
Found: C, 47.58; H, 3.12; N, 5.64

Reference Example 44

A solution of 5-aminospiro[benzo[b]furan-2(3H),1'-cyclopropane]-3-one (0.181 g.) and pyridine (0.083 mg.) in tetrahydrofuran (5 ml.) was cooled to −17°C. To the solution was added dropwise iodobenzenedichloride (0.282 g.), which had been prepared by a conventional method, dissolved in tetrahydrofuran (1.5 ml.) over 50 minutes, followed by stirring for 1 hour. The reaction mixture was poured into ice-water and extracted with ethyl acetate. The extract was washed with water, dried and the solvent was evaporated off. The residue was subjected to column-chromatography, using chloroform as the eluent. The first fraction was concentrated under reduced pressure to remove the solvent. By the above procedure, 5-amino-4-chlorospiro[benzo[b]furan-2(3H),1'-cyclopropane]-3-one was obtained as yellow crystals. Yield 0.038 g.
Mass spectrum: $C_{12}H_{12}O_2NCl$, molecular ion peak (209)

Reference Example 45

To a solution of 5-aminospiro[benzo[b]furan-2(3H),1'-cyclopropane]-3-one (2 g.) and acetone (5 ml.) in acetonitrile (100 ml.), sodium cyanoborohydride (3 g.) was added in portions. The mixture was stirred at room temperature for 38 hours, acetic acid being added occasionally to maintain the pH near neutrality. The solvent was evaporated off *in vacuo*. The resulting residue was dissolved in ether. The ether solution was washed with an aqueous solution of potassium hydroxide and then water and dried. The ether was evaporated off and the resulting oil was chromatographed on silica gel, eluting with dichloromethane. The product was recrystallized from ethyl ether-hexane to give 5-isopropylamino[benzo[b]furan-2(3H),1'-cyclopropane]-3-one (1.3 g.) as yellow prisms melting at 69—71°C.

Elemental analysis, for $C_{13}H_{15}O_2N$
Calcd.: C, 71.86; H, 6.96; N, 6.45
Found: C, 71.94; H, 7.03; N, 6.51

Reference Example 46

A mixture of 7-nitrospiro[benzo[b]furan-2(3H),1'-cyclopropane]-3-one (10 g.), formalin (37% formaldehyde, 10 ml.), trimethylamine hydrochloride (2 g.) and Raney nickel (10 g.) in ethanol (500 ml.) was subjected to reduction at 49—56°C for 3 hours under a hydrogen pressure of 50 kg/cm². After removing the catalyst by filtration, the filtrate was concentrated under reduced pressure. The concentrate was dissolved in dichloromethane and the solution was washed with a 5% aqueous solution of sodium hydrogen carbonate, then with water, followed by drying. The solvent was evaporated of and the residue was subjected to column-chromatography on silica-gel. The first fraction eluted with hexane-ethyl acetate (9:1) was recrystallized from ethanol to give 7-dimethylaminospiro[benzo[b]furan-2(3H),1'-cyclopropane]-3-one (5.87 g.) as yellow needles melting at 68.3°C.

Elemental analysis, for $C_{12}H_{13}O_2N$
Calcd.: C, 70.92; H, 6.45; N, 6.89
Found: C, 70.89; H, 6.41; N, 6.71

The second fraction was recrystallized from ethanol to give 7-methylaminospiro[benzo[b]furan-2(3H),1'-cyclopropane]-3-one (2.08 g.) as yellow needles melting at 98.2°C.

Elemental analysis, for $C_{11}H_{11}O_2N$
Calcd.: C, 69.83; H, 5.86; N, 7.40
Found: C, 70.10; H, 5.86; N, 7.36

Reference Example 47

A mixture of 7-aminospiro[benzo[b]furan-2(3H),1'-cyclopropane]-3-one (3.5 g.), 1,4-dibromobutane (4.3 g.), sodium hydrogen carbonate (1.68 g.) and N,N-dimethylformamide (50 ml.) was heated at 100°C for 4 hours. The reaction mixture was diluted with water and extracted with ethyl acetate. The extract was washed with water, dried and concentrated to remove the solvent. The residue was chromatographed on silica gel. The first fraction eluted with hexane-ethyl acetate was distilled under reduced pressure to recover yellow crystals and the product was recrystallized from ethanol to give 7-(1-pyrrolidinyl)-spiro[benzo[b]furan-2(3H),1'-cyclopropane]-3-one (0.87 g.) as yellow needles melting at 101.5°C.

Elemental analysis, for $C_{14}H_{15}O_2N$
Calcd.: C, 73.34; H, 6.59; N, 6.11
Found: C, 73.10; H, 6.52; N, 6.02

Reference Example 48

A mixture of 7-aminospiro[benzo[b]furan-2(3H),1'-cyclopropane]-3-one (1.75 g.), ethylbromide (5.45 g.) and sodium hydrogen carbonate (1.68 g.) in N,N-dimethylformamide (50 ml.) was heated at 100°C for 4 hours. The reaction mixture was diluted with water and extracted with ethyl acetate. The extract was washed with water and dried, then the solvent was evaporated off. The residue was subjected to column chromatography on silica-gel. The first fraction eluted with hexane-ethyl acetate (97:3) was recrystallized from ethanol to give 7-diethylaminospiro[benzo[b]furan-2(3H),1'-cyclopropane]-3-one (0.801 g.) as yellow needles melting at 39.3°C.

Elemental analysis, for $C_{14}H_{17}O_2N$
Calcd.: C, 72.70, H, 7.41; N, 6.06
Found: C, 72.83; H, 7.42; N, 6.11

The second fraction eluted with hexane-ethyl acetate (95:5) was recrystallized from ethanol to give 7-ethylaminospiro[benzo[b]furan-2(3H),1'-cyclopropane]-3-one (0.204 g.) as pale yellow needles melting at 80.6°C.

Elemental analysis, for $C_{12}H_{13}O_2N$
Calcd.: C, 70.92; H, 6.45; N, 6.89
Found: C, 70.71; H, 6.49; N, 6.81

Reference Example 49

$NaBH_4$ (0.9 g.) was added in portions to a well-stirred solution of 5-acetylspiro[benzo[b]furan-2(3H),1'-cyclopropane]-3-one (1 g.) in tetrahydrofuran (25 ml.) and isopropanol (3 ml.), with cooling at −50°C. The reaction mixture was then stirred at room temperature for 30 minutes, followed by dilution with ice-water, which was neutralized with aqueous ammonium chloride. The aqueous solution was extracted with ethyl acetate. The extract was washed with water and dried. The residue obtained by removal of the solvent was chromatographed on silica gel, eluting with $CHCl_3$. The product was distilled under reduced pressure to give 5-(1-hydroxyethyl)spiro[benzo[b]furan-2(3H),1'-cyclopropane]-3-one as a colourless oil. b.p. 0.05 mmHg: 110°C (bath temperature). IR$\nu_{max}^{film}$ $cm^{-1}$: 3350(OH), 1710(CO). NMR($CDCl_3$)$\delta$: 1.45(3H, d, J=6Hz, $CH_3$), 1.62(4H, q, J=3Hz, $CH_2$), 3.33(1H, b, OH), 4.83(1H, q, J=6Hz, CH), 6.97(1H, d, J=9Hz, aromat.H), 7.55(2H, m, aromat.H).

Elemental analysis, for $C_{12}H_{12}O_3$
Calcd.: C, 70.57; H, 5.92
Found: C, 70.47; H, 6.05

Reference Example 50

A mixture of $\alpha$-[(4-acetyl-2-methoxycarbonylphenyl)oxy]-$\gamma$-butyrolactone (2.8 g.), 1.8-diazabicyclo[5,4,0]-7-undecene (0.056 g.), sodium chloride (0.6 g.) and N,N'-dimethylformamide (28 ml.) was heated at 150—155°C for 4 hours. The solvent was removed *in vacuo* and the resulting residue was dissolved in ethyl acetate. The ethyl acetate solution was washed with water and dried. The residue obtained by removal of the solvent *in vacuo* was chromatographed on silica gel. The fraction eluted with dichloromethane was crystallized from ethanol to give 5-acetylspiro[benzo[b]furan-2(3H), 1'-cyclopropane]-3-one (1.15 g) as colourless crystals. IR$\nu^{KBr}_{max}$ $cm^{-1}$: 1700, 1680 (CO, $COCH_3$). NMR ($CDCl_3$)$\delta$: 1.70 (4H, q, J=7Hz, $CH_2$), 2.62 (3H, s, $COCH_3$), 7.20 (1H, d, J=9Hz, aromat.H), 8.27, 8.30 (1H, dd, J=9Hz, aromat.H), 8.29 (1H, d, J=2Hz, aromat.H). This product was identical with the product of Reference Example 31 in melting point and spectral data.

Reference Example 51

Employing $\alpha$-[(6-Acetyl-2-methoxycarbonylphenyl)-oxy]-$\gamma$-butyrolactone (3.5 g.), 1.8-diazabicyclo[5.4.0]-7-undecane (0.14 g), sodium chloride (1.1 g) and N,N-dimethylformamide (66.5 ml), a reaction was conducted in a similar manner to that in Reference Example 50. The product was re-crystallized from $CHCl_3$-hexane to give 7-acetylspiro[benzo[b]furan-2(3H), 1'-cyclopropane]-3-one (0.22 g.) as colourless needles melting at 114—115°C.

Elemental analysis, for $C_{12}H_{10}O_3$
Calcd.: C, 71.28; H, 4.99
Found: C, 71.39; H, 4.96

Example 1

5-Aminospiro[benzo[b]furan-2(3H), 1'-cyclopropane]-3-one (10 g.), ethylene oxide (5 g.) and methanol (150 ml.) were allowed to react in a sealed tube at 70°C for 16 hours, and after cooling the solvent was evaporated off under reduced pressure. The residue was recrystallized from ethyl acetate-hexane to give 5-bis($\beta$-hydroxyethyl)aminospiro[benzo[b]furan-2(3H), 1'-cyclopropane]-3-one (12.9 g.) as yellow needles melting at 96—97°C.

Elemental analysis, for $C_{14}H_{17}O_4N$
Calcd.: C, 63.86; H, 6.51; N, 5.32
Found: C, 63.75; H, 6.54; N, 5.23

The crystals obtained from the mother liquor of the above recrystallization were subjected to column-chromatography on silica-gel, and the fraction eluted with 2% ethanol-methylene chloride was recrystallized from ethyl acetate-hexane to give 5-($\beta$-hydroxyethyl)aminospiro[benzo[b]furan-2(3H), 1'-cyclopropane]-3-one (1.6 g.) as yellow needles melting at 74—75°C.

Elemental analysis, for $C_{12}H_{13}O_3N$
Calcd.: C, 65.74; H, 5.98; N, 6.39
Found: C, 65.71; H, 6.02; N, 6.23

Example 2

7-Aminospiro[benzo[b]furan-2(3H), 1'-cyclopropane]-3-one (350 mg.) was reacted in the same manner as in Example 1, and the product was subjected to column-chromatography on silica-gel. The first fraction eluted with chloroform was recrystallized from ethyl acetate-hexane to give 7-($\beta$-hydroxyethyl)-aminospiro[benzo[b]furan-2(3H), 1'-cyclopropane]-3-one as yellow needles melting at 74—77°C.

Elemental analysis, for $C_{12}H_{13}O_3N$
Calcd.: C, 65.74; H, 5.98; N, 6.39
Found: C, 65.96; H, 5.98; N, 6.19

The second fraction eluted with 2% ethanol-chloroform was recrystallized from ethyl acetate-hexane to give 7-bis($\beta$-hydroxyethyl)aminospiro[benzo[b]furan-2(3H), 1'-cyclopropane]-3-one as yellow needles melting at 74—76°C.

Elemental analysis, for $C_{14}H_{17}O_4N$
Calcd.: C, 63.86; H, 6.51; N, 5.32
Found: C, 63.67; H, 6.51; N, 5.35

Example 3

2 ml. of an aqueous solution of sodium cyanate (130 mg.) were added dropwise to a solution of 5-aminospiro[benzo[b]furan-2(3H), 1'-cyclopropane]-3-one (1.75 g.) in methanol (10 ml.) and acetic

acid (5 ml.), and the mixture was stirred for 2 hours at room temperature. Ice-water was added to the reaction mixture, and the precipitated crystals were collected by filtration and recrystallized from ethanol to give colourless needles of 5-ureidospiro[benzo[b]furan-2(3H), 1'-cyclopropane]-3-one (0.74 g.).
m.p. 250—252°C.

Elemental analysis, for $C_{11}H_{10}O_3N_2$
Calcd.: C, 60.54; H, 4.62; N, 12.84
Found: C, 60.42; H, 4.42; N, 12.94

Example 4

5-Aminospiro[benzo[b]furan-2(3H), 1'-cyclopropane]-3-one (1.75 g.) and methyl isocyanate (1 ml.) were stirred in tetrahydrofuran for 2 hours at room temperature. The solvent was evaporated off under reduced pressure, and the residue was recrystallized from ethanol to give yellow prisms of 5-methylureidospiro[benzo[b]furan-2(3H), 1'-cyclopropane]-3-one (1.06 g.).
m.p. 196—204°C.

Elemental analysis, for $C_{12}H_{12}O_3N_2$
Calcd.: C, 62.06; H, 5.21; N, 12.06
Found: C, 61.87; H, 5.11; N, 11.67

Example 5

5-Aminospiro[benzo[b]furan-2(3H), 1'-cyclopropane]-3-one (1.75 g.) and methyl isothiocyanate (1.1 g.) were stirred in acetonitrile (60 ml.) at room temperature for 15 hours, and then the mixture was refluxed under heating for 3 hours. The solvent was evaporated off under reduced pressure, and the residue was recrystallized from methanol to give yellow prisms of 5-methylthioureido-spiro[benzo[b]furan-2(3H), 1'-cyclopropane]-3-one (1.62 g.); m.p. 191—194°C.

Elemental analysis, for $C_{12}H_{12}O_2N_2S$
Calcd.: C, 58.04; H, 4.87; N, 11.28
Found: C, 58.07; H, 4.87; N, 11.00

Example 6

5-Aminospiro[benzo[b]furan-2(3H), 1'-cyclopropane]-3-one (1.75 g.) was dissolved in conc. HCl (2.5 ml.) and ice-water (20 ml.) and 2 ml. of an aqueous solution of sodium nitrite (0.7 g.) were added dropwise to the resulting solution under ice-cooling. After stirring for one hour and adding toluene (5 ml.) the solution was neutralized with sodium hydrogen carbonate at −20°C. To 18 ml of another aqueous solution of cuprous cyanide prepared from cuprous chloride (2.4 g.) and potassium cyanide (4.4 g.), ethyl acetate (20 ml.), was added, and, to the mixture, the above neutralized solution of diazonium salt was added under ice-cooling and with stirring. The mixture was stirred for half an hour at room temperature and further stirred for half an hour at 70°C. The insolubles were removed by filtration, and the filtrate was extracted with ethyl acetate. The extract was washed with an aqueous solution of sodium carbonate, diluted hydrochloric acid and water in the order mentioned, and dried. The solvent was evaporated off under reduced pressure, and the obtained residue was recrystallized from methanol to give colourless needles of 5-cyanospiro[benzo[b]furan-2(3H), 1'-cyclopropane]-3-one (1.4 g.).
m.p. 148—149°C.

Elemental analysis, for $C_{11}H_7O_2N$
Calcd.: C, 71.35; H, 3.81; N, 7.56
Found: C, 71.13; H, 3.70; N, 7.39

Example 7

5-Acetylspiro[benzo[b]furan-2(3H), 1'-cyclopropane]-3-one (13.5 g.) was added to 300 ml. of an aqueous solution of sodium hypochlorite. To the mixture was further added a small amount of a surface-active agent (polyoxyethylene octyl phenyl ether), and the mixture was stirred at 60°C for 3 hours. After cooling, the reaction mixture was diluted with water, and to the mixture a 40% aqueous solution of sodium hydrogen sulphite was added. The reaction mixture was made acidic with hydrochloric acid, and the resulting crystals were collected by filtration. After washing with water and drying, the crystals were recrystallized from ethanol to give colourless needles of 5-carboxy-spiro[benzo[b]furan-2(3H), 1'-cyclopropane]-3-one (12.68 g.) m.p. 215—228°C.

Elemental analysis, for $C_{11}H_8O_4$
Calcd.: C, 64.70; H, 3.95
Found: C, 64.75; H, 3.84

Example 8

5-Carboxyspiro[benzo[b]furan-2(3H), 1′-cyclopropane]-3-one (2 g.) and sodium hydrogen carbonate (1.25 g.) were suspended in acetone (50 ml.) and N,N-dimethylformamide (5 ml.) and, with stirring, dimethyl sulphate (2 ml.) was added dropwise to the mixture. The reaction mixture was refluxed under heating for 24 hours, and, after cooling, the solvent was evaporated off under reduced pressure. The residue was diluted with water and the solution was extracted with ethyl acetate. The extract was washed with an aqueous solution of sodium hydrogen carbonate and dried. After removing the solvent by evaporation under reduced pressure, the residue was recrystallized from methanol to give colourless needles of 5-methoxycarbonylspiro[benzo[b]furan-2(3H), 1′-cyclopropane]-3-one (1.25 g.). m.p. 89—91°C.

Elemental analysis, for $C_{12}H_{10}O_4$
Calcd.: C, 66.05; H, 4.62
Found: C, 66.19; H, 4.62

Example 9

To a solution of 5-carboxyspiro[benzo[b]furan-2(3H), 1′-cyclopropane]-3-one (1.02 g.) in 30 ml. of tetrahydrofuran triethylamine (0.5 g.) was added and, with stirring at —18°C, isobutyl chlorocarbonate (0.75 g.) was added dropwise to the solution. After stirring for 30 minutes, 2-(diethylamino)ethylamine (0.58 g.) was added and the mixture was stirred at room temperature for 3 hours. The solvent was evaporated off under reduced pressure, and the residue was dissolved in ethanol. Oxalic acid was added to the solution to form the oxalate, and, after cooling, the resulting crystals were collected by filtration to give colourless needles of 5-(2-diethylaminoethyl)carbamoyl-spiro[benzo[b]furan-2(3H), 1′-cyclopropane]-3-one oxalate (1.08 g.) m.p. 174—179°C.

Elemental analysis, for $C_{17}H_{22}O_3N_2 \cdot C_2H_2O_4$
Calcd.: C, 58.15; H, 6.17; N, 7.14
Found: C, 57.92; H, 6.27; N, 7.15

Example 10

5-Carboxyspiro[benzo[b]furan-2(3H), 1′-cyclopropane]-3-one (2.04 g.) triethylamine (1.4 ml.), isobutyl chlorocarbonate (1.5 g.) and N-ethyl-2-aminomethylpyrrolidine (1.4 g.) were allowed to react in the same manner as in Example 9. The product was converted to an oxalate by a conventional procedure and recrystallization from ethanol gave colourless prisms of 5-(1-ethyl-2-pyrrolidinyl)methyl-carbamoylspiro[benzo[b]furan-2(3H), 1′-cyclopropane]-3-one (1.567 g.). m.p. 161—166°C.

Elemental analysis, for $C_{18}H_{22}O_3N_2 \cdot C_2H_2O_4$
Calcd.: C, 59.40; H, 5.98; N, 6.93
Found: C, 59.13; H, 6.02; N, 6.75

Example 11—13

The following compounds were obtained by a procedure similar to that of Reference Example 27.

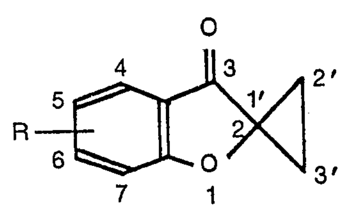

| No. | Compound R | m.p. (°C) | Elemental analysis | | |
|---|---|---|---|---|---|
| | | | Molecular formula | Upper rank: Calcd. Lower rank: Found C | H |
| 11 | 5—Ph | 116—121 | $C_{16}H_{12}O_2$ | 81.34<br>81.65 | 5.12<br>5.00 |
| 12 | 5—$SCH_3$ | 64—66 | $C_{11}H_{10}O_2S$ | 64.06<br>63.79 | 4.89<br>4.76 |
| 13 | 5—$SO_2CH_3$ | 157—159 | $C_{11}H_{10}O_4S$ | 55.45<br>55.37 | 4.23<br>4.16 |

(Ph represents phenyl)

Example 14

To a solution of 5-methylthiospiro[benzo[b]furan-2(3H), 1′-cyclopropane]-3-one (103.1 mg.) in dichloromethane (2.1 ml.), *m*-chloroperbenzoic acid (215.7 mg.) was added in small portions with stirring. After stirring for one hour, the reaction mixture was diluted with water and extracted with chloroform. The extract was washed with an aqueous solution of sodium hydrogen carbonate, an aqueous solution of sodium hydrosulphite and water in that order, and after drying the solvent was removed by evaporation under reduced pressure. The residue was recrystallized from ethanol-water to give colourless needles of 5-methylsulphonylspiro[benzo[b]furan-2(3H), 1′-cyclopropane]-3-one (85 mg.). This product was identical with the compound of Example 13 in melting point and spectral data.

Example 15

5-Methylthiospiro[benzo[b]furan-2(3H), 1′-cyclopropane]-3-one (600 mg.) and *m*-chloroperbenzoic acid (627 mg.) were allowed to react in the same manner as in Example 14. The product was recrystallized from ethyl acetate-hexane to give colourless prisms of 5-methylsulphinylspiro[benzo[b]furan-2(3H), 1′-cyclopropane]-3-one (493 mg.) m.p. 87—90°C.

Elemental analysis, for $C_{11}H_{10}O_3S$
Calcd.: C, 59.44; H, 4.54
Found: C, 59.31; H, 4.57

Example 16

A solution of 5-cyanospiro[benzo[b]furan-2(3H), 1′-cyclopropane]-3-one (0.2 g.) in 10 ml. of ethanol and 10 ml of a 2N aqueous solution of sodium hydroxide was subjected to catalytic reduction in the presence of Raney-nickel under a hydrogen stream and at an ordinary temperature and pressure. After finishing the absorption of hydrogen, the catalyst was removed by filtration, and the filtrate was diluted with water and extracted with chloroform. The extract was washed with water and, after drying, the solvent was removed by evaporation under reduced pressure. The residue was dissolved in methanol and to the solution oxalic acid was added to form the oxalate. Recrystallization from methanol-ethyl acetate gave pale brown needles of 5-aminomethylspiro[benzo[b]furan-2(3H), 1′-cyclopropane]-3-one oxalate. m.p. 192—195°C.

Elemental analysis, for $C_{11}H_{11}O_2N{\cdot}C_2H_2O_4$
Calcd.: C, 55.91; H, 4.69; N, 5.02
Found: C, 55.74; H, 4.60; N, 4.93

Example 17

5.5 ml. of a 7% aqueous solution of hydrogen peroxide and 3.7 ml. of ethanol were added dropwise to a solution of 5-cyanospiro[benzo[b]furan-2(3H), 1′-cyclopropane]-3-one (185 mg.) in ethanol (10 ml.). Then, a 2N aqueous solution of sodium hydroxide was added to the mixture to adjust a pH of 10 and the solution was stirred at 60°C for 2 hours. After cooling, the reaction mixture was neutralized with dilute hydrochloric acid and diluted with ice-water. The precipitating crystals were collected by filtration, washed with water and recrystallized from ethanol to give colourless needles of 5-carbamoylspiro[benzo[b]furan-2(3H), 1′-cyclopropane]-3-one (106 mg.). m.p. 243—244°C.

Elemental analysis, for $C_{11}H_9O_3N$
Calcd.: C, 65.02; H, 4.46; N, 6.89
Found: C, 65.19; H, 4.39; N, 6.78

## Example 18

7-Acetylspiro[benzo[b]furan-2(3H), 1'-cyclopropane]-3-one (86 mg.) was allowed to react in the same manner as in Example 7. The product was recrystallized from ethanol to give colourless needles of 7-carboxyspiro[benzo[b]furan-2(3H), 1'-cyclopropane]-3-one. m.p. 259—262°C (decomp.). Sublimes at 183°C. IR$\nu_{max}^{KBr}$ $cm^{-1}$: 1730 (COOH), 1680 (CO). NMR($CD_3OD$)$\delta$: 1.67(2H, t, J=3Hz, $CH_2$), 1.93 (2H, t, J=3Hz, $CH_2$), 7.37 (1H, t, J=8Hz, 5-aromat.H), 8.02 (1H, dd, J=2Hz, 8Hz, 4- or 6-aromat.H), 8.40 (1H, dd, J=2Hz, 8Hz, 4- or 6-aromat.H).

Calcd.: C, 64.70; H, 3.95
Found: C, 64.53; H, 4.00

## Example 19

Diethyl sulphate (5 ml.) was added dropwise to a stirred mixture of 5-carboxyspiro[benzo[b]furan-2(3H), 1'-cyclopropane]-3-one (5.1 g.) and potassium carbonate (4.32 g.) in N,N-dimethylformamide (25 ml.). The reaction mixture was heated at 60°C for 0.5 hours. After removal of the solvent *in vacuo*, the residue was poured into ice-water. The crystals separated were collected by filtration, washed with water and recrystallized from ethanol to give 5-ethoxycarbonylspiro[benzo[b]furan-2(3H), 1'-cyclopropane]-3-one (5.3 g.) as colourless needles melting at 97—98°C.

Elemental analysis, for $C_{13}H_{12}O_4$
Calcd.: C, 67.23; H, 5.21
Found: C, 67.40; H, 5.24

*Examples of preparations ready for administration*

When the compound of this invention is intended for use as an antiulcer agent, types of the preparation can be exemplified as follows.

1. Tablets

| | | |
|---|---|---|
| (1) | 5-Carboxyspiro[benzo[b]furan-2(3H), 1'-cyclopropane]-3-one | 50 g. |
| (2) | Lactose | 50 g. |
| (3) | Corn-starch | 29 g. |
| (4) | Magnesium stearate | 1 g. |
| | 1000 tablets | 130 g. |

(1), (2) and 17 g. of the corn-starch were granulated together with a paste prepared from 7 g. of the corn-starch. To these granules 5 g. of the corn-starch and (4) were added, and the mixture was compressed by a tabletting machine to prepare 1000 tablets of 7 mm. diameter, each containing 50 mg. of (1).

2. Capsules

| | | |
|---|---|---|
| (1) | 5-Bis($\beta$-hydroxyethyl)aminospiro [benzo[b]furan-2(3H),1'-cyclopropane]-3-one | 50 g. |
| (2) | Lactose | 100 g. |
| (3) | Cellulose fine powder | 45 g. |
| (4) | Magnesium stearate | 5 g. |
| | 1000 capsules | 200 g. |

All the materials were mixed and filled into 1000 capsules (gelatin capsule No. 3 defined in Japanese Pharmacopoeia 8th edition) to prepare capsules each containing 50 mg. of (1).

**Claims**

1. A spirobenzofuranone compound of the formula:

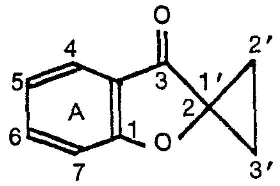

(I)

wherein Ring A represents a benzene ring which is substituted by one to four of carboxyl, $C_{2-6}$ alkoxycarbonyl, carbamoyl, $C_{1-4}$ alkylcarbamoyl, di-$C_{1-4}$ alkylamino-$C_{1-4}$ alkylcarbamoyl, N—$C_{1-4}$ alkylpyrrolidinyl-$C_{1-4}$ alkylcarbamoyl, ureido, $C_{1-4}$ alkylureido, thioureido, $C_{1-4}$ alkylthioureido, $C_{1-4}$ alkylthio, $C_{1-4}$ alkylsulphinyl, $C_{1-4}$ alkylsulphonyl, mono- or bis-($\beta$-hydroxyethyl)amino, aminomethyl, mono- or di-$C_{1-4}$ alkylaminomethyl, cyano and phenyl,

or a pharmaceutically acceptable salt thereof.

2. A compound according to claim 1, wherein the benzene ring is substituted at the 5- or 7-position on the ring.

3. A compound according to claim 1 or 2, wherein the substituent on the ring is carboxyl, $C_{1-4}$ alkylsulphonyl or mono- or bis-($\beta$-hydroxyethyl)amino.

4. A compound according to claim 3, wherein the substituent is carboxyl or methylsulphonyl.

5. A compound according to claim 1, which is 5-bis-($\beta$-hydroxyethyl)aminospiro[benzo[b]furan-2(3H), 1'-cyclopropan]-3-one.

6. A compound according to claim 1, which is 5-($\beta$-hydroxyethyl)aminospiro[benzo[b]furan-2(3H), 1'-cyclopropan]-3-one.

7. A compound according to claim 1, which is 7-bis-($\beta$-hydroxyethyl)aminospiro[benzo[b]furan-2(3H), 1'-cyclopropan]-3-one.

8. A compound according to claim 1, which is 7-($\beta$-hydroxyethyl)aminospiro[benzo[b]furan-2(3H), 1'-cyclopropan]-3-one.

9. A compound according to claim 1, which is 5-carboxyspiro[benzo[b]furan-2(3H), 1'-cyclopropan]-3-one.

10. A compound according to claim 1, which is 7-carboxyspiro[benzo[b]furan-2(3H), 1'-cyclopropan]-3-one.

11. A compound according to claim 1, which is 5-methylsulphonylspiro[benzo[b]furan-2(3H), 1'-cyclopropan]-3-one.

12. A pharmaceutical composition which comprises, as an active ingredient, an effective amount of a compound or its salt as defined in any of claims 1 to 11, and a pharmaceutically acceptable carrier or diluent therefor.

13. A compound or its salt as defined in any of claims 1 to 11 for use in the management of peptic ulcer in a mammal.

14. A process for producing a spirobenzofuranone compound as defined in claim 1, which comprises decarboxylating a compound of the formula:

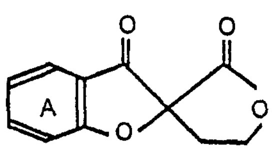

wherein Ring A is as defined in claim 1.

15. A process for producing a spirobenzofuranone compound (I) as defined in claim 1 which comprises converting at least one of the substituents of a compound of the formula:

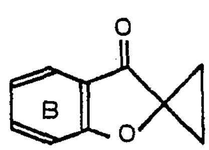

(III)

wherein Ring B represents a benzene ring which is substituted by one to four of amino, acetyl, halogen, carboxyl, $C_{2-6}$ alkoxycarbonyl, carbamoyl, $C_{1-4}$ alkylcarbamoyl, ureido, $C_{1-4}$ alkylureido thioureido, $C_{1-4}$ alkylthioureido, $C_{1-4}$ alkylthio, $C_{1-4}$ alkylsulphinyl, ($\beta$-hydroxyethyl)amino, aminomethyl and cyano,

(a) by subjecting a compound (III) wherein at least one of the substituents on Ring B is amino to alkylation with ethylene chlorohydrin or ethylene oxide to obtain a compound (I) wherein at least one of

the substituents on Ring A is mono- or bis-(β-hydroxyethyl)amino, or

(b) by subjecting a compound (III) wherein at least one of the substituents on Ring B is acetyl to a haloform reaction with hypohalogenite, or by subjecting a pyridinium salt obtained from the acetyl-substituted compound (III) with halogen and pyridine to hydrolysis, or by subjecting a compound (III) wherein at least one of the substituents on Ring B is cyano to hydrolysis, to obtain a compound (I) wherein at least one of the substituents on Ring A is carboxyl, or

(c) by subjecting a compound (III) wherein at least one of the substituents on Ring B is carboxyl to esterification with alkyl halogenide or di-alkylsulphuric acid in the presence of alkali metal hydrogen carbonate or alkali metal carbonate to obtain a compound (I) wherein at least one of the substituents on Ring A is $C_{2-6}$ alkoxycarbonyl, or

(d) by subjecting a compound (III) wherein at least one of the substituents on Ring B is cyano to hydrolysis with a base in the presence of dimethyl sulphoxide or to a reaction with hydrogen peroxide under alkaline conditions, or by subjecting a compound (III) wherein at least one of the substituents on Ring B is carboxyl or a reactive derivative thereof to condensation with an amine, to obtain a compound (I) wherein at least one of the substituents on Ring A is carbamoyl, $C_{1-4}$ alkylcarbamoyl, di-$C_{1-4}$ alkylamino-$C_{1-4}$ alkylcarbamoyl or N—$C_{1-4}$ alkylpyrrolidinyl-$C_{1-4}$ alkylcarbamoyl, or

(e) by subjecting a compound (III) wherein at least one of the substituents on Ring B is amino to a reaction with cyanic acid, isocyanic acid ester, thiocyanic acid or isothiocyanic acid ester, to obtain a compound (I) wherein at least one of the substituents on Ring A is ureido, $C_{1-4}$ alkylureido, thioureido or $C_{1-4}$ alkylthioureido, or

(f) by subjecting a compound (III) wherein at least one of the substituents on Ring B is halogen or a diazonium salt derived from an amino-substituted compound (III), to a reaction with a metal cyanide, to obtain a compound (I) wherein at least one of the substituents on Ring A is cyano.

**Revendications**

1. Composé spirobenzofuranone de formule:

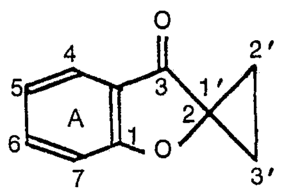

(I)

où le noyau A représente un noyau benzénique qui est substitué par un à quatre carboxyle, $C_{2-6}$alcoxycarbonyle, carbamoyle, $C_{1-4}$alkyl-carbamoyle, di-$C_{1-4}$alkylamino-$C_{1-4}$alkyl-carbamoyle, N—$C_{1-4}$alkylpyrrolidinyl-$C_{1-4}$alkyl-carbamoyle, uréido, $C_{1-4}$alkyluréido, thiouréido, $C_{1-4}$alkyl-thiouréido, $C_{1-4}$alkylthio, $C_{1-4}$alkyl-sulphinyle, $C_{1-4}$alkylsulphonyle, mono- ou bis-(β-hydroxyéthyl)amino, aminométhyle, mono- ou di-$C_{1-4}$alkylaminométhyle, cyano et phényle, ou un sel pharmaceutiquement acceptable de ce composé.

2. Composé selon la revendication 1, où le noyau benzénique est substitué en position 5 ou 7 du noyau.

3. Composé selon les revendications 1 ou 2, où le substituant sur le noyau est carboxyle, $C_{1-4}$alkylsulphonyle ou mono- ou bis-(β-hydroxyéthyl)amino.

4. Composé selon la revendication 3, où le substituant est carboxyle ou méthylsulphonyle.

5. Composé selon la revendication 1, qui est la 5-bis-(β-hydroxyéthyl)aminospiro[benzo[b]furane-2(3H), 1′-cyclopropane]-3-one.

6. Composé selon la revendication 1, qui est la 5-(β-hydroxyéthyl)aminospiro[benzo[b]furane-2(3H), 1′-cyclopropane]-3-one.

7. Composé selon la revendication 1, qui est la 7-bis-(β-hydroxyéthyl)aminospiro[benzo[b]furane-2(3H), 1′-cyclopropane]-3-one.

8. Composé selon la revendication 1, qui est la 7-(β-hydroxyéthyl)aminospiro[benzo[b]furane-2(3H), 1′-cyclopropane]-3-one.

9. Composé selon la revendication 1, qui est la 5-carboxyspiro[benzo[b]furane-2(3H), 1′-cyclopropane]-3-one.

10. Composé selon la revendication 1, qui est la 7-carboxyspiro[benzo[b]furane-2(3H), 1′-cyclopropane]-3-one.

11. Composé selon la revendication 1, qui est la 5-méthylsulphonylspiro[benzo[b]furane-2(3H), 1′-cyclopropane]-3-one.

12. Composition pharmaceutique contenant comme produit actif, une quantité efficace d'un compose ou de son sel tel que défini dans l'une quelconque des revendications 1 à 11, ainsi qu'un véhicule ou diluant pharmaceutiquement acceptable pour ce composé.

13. Composé ou son sel tel que défini dans l'une quelconque des revendications 1 à 11, à utiliser

pour le traitement d'un ulcère gastrique chez un mammifère.

14. Procédé de préparation d'un composé spirobenzofuranone tel que défini dans la revendication 1, comprenant la décarboxylation d'un composé de formule:

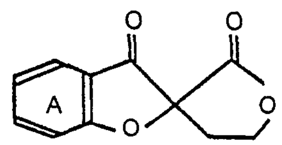

où le noyau A est tel qui défini dans la revendication 1.

15. Procédé de préparation d'un composé spirobenzofuranone (I) tel que défini dans la revendication 1, comprenant la transformation d'au moins l'un des substituants d'un composé de. formule:

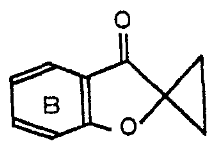

(II)

où le noyau B représente un noyau benzénique qui est substitué par 1 à 4 amino, acétyle, halogène, carboxyle, $C_{2-6}$alcoxycarbonyle, carbamoyle, $C_{1-4}$alkylcarbamoyle, uréido, $C_{1-4}$alkyluréido, thiouréido, $C_{1-4}$alkylthiouréido, $C_{1-4}$alkylthio, $C_{1-4}$alkylsulphinyle, ($\beta$-hydroxyéthyl)amino, aminométhyle et cyano,

(a) en soumettant un composé (III) où au moins un des substituants sur le noyau B est amino, à l'alkylation avec la chlorhydrine d'éthylène ou l'oxyde d'éthylène pour obtenir un composé (I) où au moins l'un des substituants sur le noyau A est mono- ou bis-($\beta$-hydroxyéthyl)amino, ou

(b) en soumettant un composé (III) où au moins l'un des substituants sur le noyau B est acétyle à une réaction halogénoforme avec un hypohalogénure, ou en soumettant un sel de pyridinium obtenu à partir du composé (III) acétyle substitué, d'un halogène et pyridine, à l'hydrolyse, ou en soumettant un composé (III) où au moins l'un des substituants sur le noyau B est cyano à l'hydrolyse, pour obtenir un composé (I) où au moins l'un des substituants sur le noyau A est carboxyle, ou

(c) en soumettant un composé (III) où au moins l'un des substituants sur le noyau B est carboxyle à l'estérification avec un halogénure d'alkyle ou avec l'acide di-alkylsulfurique, en présence d'un carbonate acide de métal alcalin ou d'un carbonate de métal alcalin pour obtenir un composé (I) où au moins l'un des substituants sur le noyau A est un $C_{2-6}$alcoxycarbonyle, ou

(d) en soumettant un composé (III) où au moins l'un des substituants sur le noyau B est cyano à l'hydrolyse avec une base, en présence de diméthylsulfoxyde, ou à une réaction avec l'eau oxygénée dans des conditions alcalines, ou en soumettant un composé (III) où au moins l'un des substituants sur le noyau B est carboxyle ou un dérivé réactif de ce dernier à la condensation avec une amine, pour obtenir un composé (I) où au moins l'un des substituants sur le noyau A est carbamoyle, $C_{1-4}$alkylcarbamoyle, di-$C_{1-4}$alkylamino-$C_{1-4}$alkylcarbamoyle ou N—$C_{1-4}$alkyl-pyrrolidinyle-$C_{1-4}$alkylcarbamoyle, ou

(e) en soumettant un composé (III) où au moins l'un des substituants sur le noyau B est amino à une réaction avec l'acide cyanique, avec un ester de l'acide isocyanique, l'acide thiocyanique ou un ester de l'acide isothiocyanique, pour obtenir un composé (I) où au moins l'un des substituants sur le noyau A est uréido, $C_{1-4}$alkyluréido, thiouréido ou $C_{1-4}$alkylthiouréido, ou

(f) en soumettant un composé (III) où au moins l'un des substituants sur le noyau B est un halogène ou un sel diazonium dérivé d'un composé (III) amino-substitué, à une réaction avec un cyanure métallique, pour obtenir un composé (I) où au moins l'un des substituants sur le noyau A est cyano.

**Patentansprüche**

1. Spirobenzofuranon-Verbindung der Formel (I)

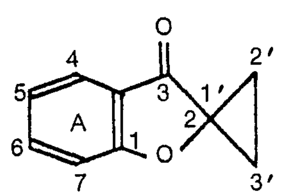

(I)

in der der Ring A einen Benzol-Ring bezeichnet, der durch eine bis vier Gruppen ausgewählt aus Car-

boxyl, $C_{2-6}$-Alkoxycarbonyl, Carbamoyl, $C_{1-4}$-Alkylcarbamoyl, Di-$C_{1-4}$-alkylamino-$C_{1-4}$-alkylcarbamoyl, N—$C_{1-4}$-Alkylpyrrolidinyl-$C_{1-4}$-alkylcarbamoyl, Ureido, $C_{1-4}$-Alkylureido, Thioureido, $C_{1-4}$-Alkylthioureido, $C_{1-4}$-Alkylthio, $C_{1-4}$-Alkylsulfinyl, $C_{1-4}$-Alkylsulfonyl, Mono- oder Bis($\beta$-hydroxyethyl)amino, Aminomethyl, Mono- oder Di-$C_{1-4}$-alkylaminomethyl, Cyano und Phenyl substituiert ist,

oder ein pharmazeutisch unbedenkliches Salz derselben.

2. Verbindung nach Anspruch 1, dadurch gekennzeichnet, daß der Benzol-Ring in der 5- oder 7-Stellung an dem Ring substituiert ist.

3. Verbindung nach Anspruch 1 oder 2, dadurch gekennzeichnet, daß der Substituent an dem Ring Carboxyl, $C_{1-4}$-Alkylsulfonyl oder Mono- oder Bis($\beta$-hydroxyethyl)amino ist.

4. Verbindung nach Anspruch 3, dadurch gekennzeichnet, daß der Substituent Carboxyl oder Methylsulfonyl ist.

5. Verbindung 5-Bis($\beta$-hydroxyethyl)aminospiro[benzo[b]furan-2(3H),1'-cyclopropan]-3-on nach Anspruch 1.

6. Verbindung 5-($\beta$-Hydroxyethyl)aminospiro[benzo[b]furan-2(3H),1'-cyclopropan]-3-on nach Anspruch 1.

7. Verbindung 7-Bis($\beta$-hydroxyethyl)aminospiro[benzo[b]furan-2(3H),1'-cyclopropan]-3-on nach Anspruch 1.

8. Verbindung 7-($\beta$-Hydroxyethyl)aminospiro[benzo[b]furan-2(3H),1'-cyclopropan]-3-on nach Anspruch 1.

9. Verbindung 5-Carboxyspiro[benzo[b]furan-2(3H),1'-cyclopropan]-3-on nach Anspruch 1.

10. Verbindung 7-Carboxyspiro[benzo[b]furan-2(3H),1'-cyclopropan]-3-on nach Anspruch 1.

11. Verbindung 5-Methylsulfonylspiro[benzo[b]furan-2(3H),1'-cyclopropan]-3-on nach Anspruch 1.

12. Pharmazeutische Zusammensetzung, enthaltend als Wirkstoff eine wirksame Menge einer Verbindung nach Anspruch 1 bis 11 oder eines Salzes derselben und ein pharmazeutisch unbedenkliches Trägermaterial oder Verdünnungsmittel für diesen.

13. Verbindung nach Anspruch 1 bis 11 oder deren Salz zur Verwendung bei der Behandlung von peptischem Ulcer bei Säugern.

14. Verfahren zur Herstellung einer Spirobenzofuranon-Verbindung nach Anspruch 1, dadurch gekennzeichnet, daß eine Verbindung der Formel

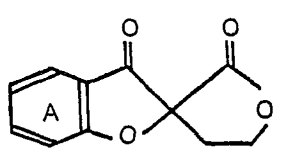

in der der Ring A die in Anspruch 1 bezeichnete Bedeutung hat, decarboxyliert wird.

15. Verfahren zur Herstellung einer Spirobenzofuranon-Verbindung (I) nach Anspruch 1, dadurch gekennzeichnet, daß wenigstens einer der Substituenten einer Verbindung der Formel (III)

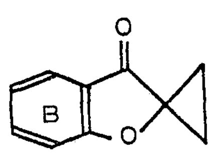

(III)

in der der Ring B einen Benzol-Ring bezeichnet, der durch eine bis vier Gruppen ausgewählt aus Amino, Acetyl, Halogeno, Carboxyl, $C_{2-6}$-Alkoxycarbonyl, Carbamoyl, $C_{1-4}$-Alkylcarbamoyl, Ureido, $C_{1-4}$-Alkylureido, Thioureido, $C_{1-4}$-Alkylthioureido, $C_{1-4}$-Alkylthio, $C_{1-4}$-Alkylsulfinyl, ($\beta$-Hydroxyethyl)amino, Aminomethyl und Cyano substituiert ist,

dadurch umgewandelt wird,

(a) daß eine Verbindung (III), in der wenigstens einer der Substituenten am Ring B Amino ist, einer Alkylierung mit Ethylenchlorhydrin oder Ethylenoxid unterworfen wird, wodurch eine Verbindung (I) erhalten wird, in der wenigstens einer der Substituenten am Ring A Mono- oder Bis-($\beta$-hydroxyethyl)amino ist, oder

(b) daß eine Verbindung (III), in der wenigstens einer der Substituenten am Ring B Acetyl ist, einer Haloform-Reaktion mit einem Hypohalogenit unterworfen wird oder ein aus der acetylsubstituierten Verbindung (III) mit Halogen und Pyridin erhaltenes Pyridinium-Salz der Hydrolyse unterworfen wird oder eine Verbindung (III), in der wenigstens einer der Substituenten am Ring B Cyano ist, der Hydrolyse unterworfen wird, wodurch eine Verbindung (I) erhalten wird, in der wenigstens einer der Substituenten am Ring A Carboxyl ist, oder

(c) daß eine Verbindung (III), in der wenigstens einer der Substituenten am Ring B Carboxyl ist, der

Veresterung mit einem Alkylhalogenid oder mit Dialkylschwefelsäure in Gegenwart eines Alkalimetallhydrogencarbonats oder Alkalimetallcarbonats unterworfen wird, wodurch eine Verbindung (I) erhalten wird, in der wenigstens einer der Substituenten am Ring A $C_{2-6}$-Alkoxycarbonyl ist, oder

(d) daß eine Verbindung (III), in der wenigstens einer der Substituenten am Ring B Cyano ist, der Hydrolyse mit einer Base in Gegenwart von Dimethylsulfoxid oder einer Reaktion mit Hydrogenperoxid unter alkalischen Bedindungen unterworfen wird oder eine Verbindung (III), in der wenigstens einer der Substituenten am Ring B Carboxyl oder davon abgeleitetes reaktionsfähiges Derivat ist, der Kondensation mit einem Amin unterworfen wird, wodurch eine Verbindung (I) erhalten wird, in der wenigstens einer der Substituenten am Ring A Carbamoyl, $C_{1-4}$-Alkylcarbamoyl, Di-$C_{1-4}$-alkylamino-$C_{1-4}$-alkylcarbamoyl oder N—$C_{1-4}$-Alkylpyrrolidinyl-$C_{1-4}$-alkylcarbamoyl ist, oder

(e) daß eine Verbindung (III), in der wenigstens einer der Substituenten am Ring B Amino ist, einer Reaktion mit Cyansäure, Isocyansäureester, Thiocyansäure oder Isothiocyansäureester unterworfen wird, wodurch eine Verbindung (I) erhalten wird, in der wenigstens einer der Substituenten am Ring A Ureido, $C_{1-4}$-Alkylureido, Thioureido oder $C_{1-4}$-Alkylthioureido ist, oder

(f) daß eine Verbindung (III), in der wenigstens einer der Substituenten am Ring B Halogen oder ein von einer aminosubstituierten Verbindung (III) abgeleitetes Diazoniumsalz ist, einer Reaktion mit einem Metallcyanid unterwirft, wodurch eine Verbindung (I) erhalten wird, in der wenigstens einer der Substituenten am Ring A Cyano ist.